Europäisches Patentamt

European Patent Office

Office européen des brevets

(11) Numéro de publication: **0 219 377**
**A1**

## (12) DEMANDE DE BREVET EUROPEEN

(21) Numéro de dépôt: **86401942.7**

(22) Date de dépôt: **04.09.86**

(51) Int. Cl.4: **A 61 K 31/165**
**A 61 K 9/16**

(30) Priorité: **05.09.85 FR 8513210**

(43) Date de publication de la demande:
**22.04.87 Bulletin 87/17**

(84) Etats contractants désignés:
**AT BE CH DE FR GB IT LI LU NL SE**

(71) Demandeur: **RHONE-POULENC SANTE**
**Les Miroirs 18 Avenue d'Alsace**
**F-92400 Courbevoie Cedex (FR)**

(72) Inventeur: **Proton, Hubert**
**100 Waverley Appt. 6**
**Ottawa (Ontario)K2P OV2 (CA)**

(74) Mandataire: **Pilard, Jacques et al**
**RHONE-POULENC RECHERCHES Service Brevets**
**Pharma 25, Quai Paul Doumer**
**F-92408 Courbevoie Cedex (FR)**

(54) Procédé de préparation d'agglomerats sphériques de paracétamol et produits obtenus.

(57) Procédé de préparation d'agglomérats sphériques de paracétamol caractérisé en ce que l'on agite une suspension épaisse de paracétamol dans l'eau en présence d'un liquide liant non miscible à l'eau choisi parmi les acétates aliphatiques comportant au moins 5 atomes de carbone, les alcools aliphatiques comportant au moins 5 atomes de carbone et les cétones aliphatiques ou cyclaniques comportant au moins 5 atomes de carbone.

EP 0 219 377 A1

# 0 219 377

**Description**

## PROCEDE DE PREPARATION D'AGGLOMERATS SPHERIQUES DE PARACETAMOL ET PRODUITS OBTENUS

La paracétamol est un des analgésiques les plus importants actuellement utilisés. Toutefois la mise sous forme pharmaceutique du produit industriel est rendue difficile du fait que le paracétamol cristallisé qui a une bonne coulabilité, se dissout lentement dans l'eau tandis que les produits broyés utilisés dans les spécialités pharmaceutiques qui se dissolvent rapidement ont une coulabilité médiocre.

Il a maintenant été trouvé, et c'est l'objet de la présente invention, que l'on peut préparer des agglomérats sphériques de paracétamol qui ne présentent ni les inconvénients du produit cristallisé ni ceux du produit broyé.

Le procédé selon l'invention est caractérisé en ce que l'on prépare des agglomérats sphériques de paracétamol par agitation d'une suspension épaisse de paracétamol dans l'eau en présence d'un liquide liant non miscible à l'eau choisi parmi les acétates aliphatiques comportant au moins 5 atomes de carbone, les alcools aliphatiques comportant au moins 5 atomes de carbone et les cétones aliphatiques ou cycliques comportant au moins 5 atomes de carbone.

Les acétates aliphatiques utilisés sont en particulier les acétates de propyle, isopropyle, butyle, pentyle, isopentyle et de préférence l'acétate d'isobutyle. Les alcools aliphatiques utilisés sont en particulier l'isopentanol et l'octanol. Les cétones utilisées sont en particulier la méthylisobutylcétone et la cyclohexanone.

Le paracétamol de départ peut être du produit cristallisé ou broyé.

La quantité d'eau par rapport au paracétamol n'est pas critique ; toutefois, en raison de la solubilité non négligeable du paracétamol dans l'eau, on préfère utiliser des suspensions épaisses correspondant à environ 300 à 500 cm3 d'eau pour 100 g de paracétamol.

La vitesse d'agitation du mélange doit être suffisante pour obtenir une suspension homogène du paracétamol dans l'eau et une dispersion homogène du liquide liant, sans toutefois provoquer la formation d'émulsions. Une vitesse de 500 à 700 t/min est généralement utilisée lorsque l'on opère dans un réacteur de 1 litre.

La quantité de liquide liant par rapport au paracétamol est critique. En dessous d'une quantité minimale de liquide liant l'agglomération ne se produit pas ; en revanche si l'on utilise une trop grande quantité de liquide liant les agglomérats ont tendance à se désagréger. Pour chaque liquide liant la détermination des proportions entre lesquelles les agglomérats sphériques se forment et restent stables peut être effectuée de la manière suivante. A une suspension de 100 g de paracétamol dans 300 cm3 d'eau maintenue sous agitation énergique (vitesse de rotation 700 t/min) on ajoute goutte à goutte le liquide liant. Des parties aliquotes de la suspension sont prélevées au cours de l'addition dont l'examen permet de déterminer la quantité minimale de liquide liant nécessaire à la formation des agglomérats sphériques. L'addition du liquide liant est poursuivie pour déterminer à partir de quelle quantité dudit liquide les agglomérats sphériques commencent à se désagréger. Selon la nature exacte du liquide liant la quantité minimale de celui-ci à utiliser varie globalement entre 0,4 et 0,6 g par gramme de paracétamol ainsi que le montre le tableau suivant :

| Liquide liant | Quantité minimale g de liquide liant par g de paracétamol |
|---|---|
| Acétate d'isopropyle | 0,53 |
| Acétate d'isobutyle | 0,5 |
| Acétate de propyle | 0,4 |
| Acétate de butyle | 0,4 |
| Acétate d'isopentyle | 0,4 |
| Acétate de pentyle | 0,4 |
| Méthylisobutylcétone | 0,44 |

Lors de la mise en oeuvre du procédé selon l'invention il n'est pas nécessaire d'ajouter progressivement le liquide liant ; la quantité de celui-ci nécessaire pour la formation des agglomérats sphériques peut être ajoutée

2

en une seule fois à la suspension aqueuse agitée de paracétamol. D'une manière générale la formation des agglomérats sphériques se produit quelques minutes après l'addition du liquide liant. Le diamètre des agglomérats croît ensuite à peu près linéairement par rapport à la durée de l'agitation puis reste pratiquement constant. Ainsi, dans le cas de l'acétate d'isobutyle (0,55 g par g de paracétamol) les agglomérats ont un diamètre du 450 $\mu$ après 15 minutes d'agitation ; ce diamètre croît linéairement pendant les 90 minutes suivantes pour atteindre 900 $\mu$ ; après une durée totale d'agitation de 2 heures 45 minutes le diamètre des agglomérats est de 1000 $\mu$ et reste inchangé lorsque l'agitation est poursuivie pendant encore 2 heures. Il est donc possible d'arrêter la croissance des agglomérats lorsque ceux-ci ont atteint la dimension désirée.

Les agglomérats sphériques peuvent être séparés du milieu de formation par toute méthode physico-chimique habituelle, en particulier par filtration. Les constituants (eau et liquide liant) de la phase liquide peuvent être séparés, par exemple par distillation azéotropique, et recyclés dans des opérations ultérieures.

Les agglomérats sphériques obtenus selon le procédé de l'invention, et qui constituent eux-mêmes un autre objet de l'invention, présentent des propriétés intéressantes de coulabilité, de dissolution et de compressibilité.

Les exemples suivants, donnés à titre non limitatif, montrent comment l'invention peut être mise en pratique.

EXEMPLE 1

Dans un réacteur on introduit et agite à 700 t/min un mélange de 100 g de paracétamol broyé super fin (refus à 50 < 10 %, refus à 80 $\mu$ < 1 %), 300 g d'eau et 50 g d'acétate d'isobutyle. Sous l'effet de l'agitation il se forme rapidement des agglomérats sphériques. Après une durée d'agitation comprise entre 15 et 90 minutes selon la taille désirée des agglomérats (300 à 1000 environ), la suspension est filtrée et le gâteau lavé avec 1000 cm3 d'eau. Les agglomérats sont séchés sous pression réduite (50 torr ; 6,6 kPa) à 60°C pendant 12 heures. On obtient ainsi 77,4 g de paracétamol sous forme d'agglomérats sphériques.

Les propriétés de coulabilité et de dissolution du produit ainsi obtenu sont comparées à celle de paracétamol cristallisé

paracétamol broyé (refus à 425 $\mu$ < 0,5 %, refus à 250 $\mu$ < 5 %)

paracétamol broyé super fin (refus à 50 $\mu$ < 10 %, refus à 80 $\mu$ < 1 %)

a) coulabilité : les produits sont testés sur le combiné Hosokawa commercialisé par Hosokawa Europe Ltd, St John's Estate, Tylers Green High Wycombe, Buckinghamshire, England. Les résultats suivants sont obtenus :

| Produit | Angle de talus | Angle de spatule | Densité (non tassée) |
|---|---|---|---|
| Aggloméré | 37° | 41° | 400 kg/m3 |
| Cristallisé | 39° | 39° | 700 kg/m3 |
| Broyé | 56° | 74° | 280 kg/m3 |
| Broyé super fin | 60° | 76° | 150 kg/m3 |

Le produit aggloméré selon l'invention a une coulabilité aussi bonne que le produit cristallisé, même légèrement meilleure du point de vue dynamique et très supérieure à celles des deux produits broyés.

b) dissolution : pour chaque produit on prépare 3 suspensions respectivement de 1, 2 et 3 g dans 200 cm3 d'eau ; ces suspensions sont agitées à température ambiante jusqu'à dissolution totale du solide, l'agitation étant de toute façon arrêtée au bout de 10 minutes et l'insoluble séparé par filtration, séché et pesé. Les résultats suivants sont obtenus :

| Produit | Concentration 5 g/l | | Concentration 10 g/l | | Concentration 15 g/l | |
|---|---|---|---|---|---|---|
| | Temps de dissolution | Pourcentage dissous | Temps de dissolution | Pourcentage dissous | Temps de dissolution | Pourcentage dissous |
| Aggloméré | 4 mn 30 | 100 | 7 mn | 100 | 10 mn | > 98 |
| Cristallisé | 10 mn | > 95 | 10 mn | 85 | 10 mn | 83 |
| Broyé | 3 mn | 100 | 6 mn | 100 | 10 mn | > 98 |
| Broyé super fin | 2 mn | 100 | 4 mn | 100 | 10 mn | > 98 |

Le produit aggloméré selon l'invention se dissout dans l'eau aussi facilement que les produits broyés.

EXEMPLE 2

En opérant comme à l'exemple 1, mais à partir de 100 g de paracétamol, 500 g d'eau et 50 g d'acétate d'isobutyle, on obtient 72 g d'agglomérats sphériques.

EXEMPLES 3 à 7

En opérant comme à l'exemple 1, mais à partir de 100 g de paracétamol et avec divers liquides liant, on obtient pareillement des agglomérats sphériques.

| Exemple | Liquide liant | Poids de liquide liant (g) | Volume d'eau (cm3) | Poids d'agglomérats |
|---|---|---|---|---|
| 3 | Acétate d'isopropyle | 53 | 300 | env. 70 g |
| 4 | Acétate de propyle | 40 | 300 | env. 70 g |
| 5 | Acétate de butyle | 40 | 300 | env. 70 g |
| 6 | Acétate d'isopentyle | 40 | 300 | env. 70 g |
| 7 | Acétate de pentyle | 40 | 500 | env. 70 g |

EXEMPLE 8

En opérant comme à l'exemple 1, mais à partir de 100 g de paracétamol, 300 g d'eau et 44 g de méthylisobutylcétone, on obtient des résultats analogues à ceux obtenus dans les exemples précédents.

**Revendications**

1 - Procédé de préparation d'agglomérats sphériques de paracétamol caractérisé en ce que l'on agite une suspension épaisse de paracétamol dans l'eau en présence d'un liquide liant non miscible à l'eau choisi parmi les acétates aliphatiques comportant au moins 5 atomes de carbone, les alcools aliphatiques comportant au moins 5 atomes de carbone et les cétones aliphatiques ou cyclaniques comportant au moins 5 atomes de carbone.

2 - Procédé selon la revendication 1 caractérisé en ce que l'acétate aliphatique utilisé est choisi parmi les acétates de propyle, isopropyle, butyle, pentyle et isopentyle.

3 - Procédé selon la revendication 1 caractérisé en ce que l'alcool aliphatique utilisé est choisi parmi l'isopentanol et l'octanol.

4 - Procédé selon la revendication 1 caractérisé en ce que la cétone utilisée est choisie parmi la méthylisobutylcétone et la cyclohexanone.

5 - Agglomérats sphériques obtenus par le procédé selon l'une des revendications 1 à 4.

4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-2 221 178  (CIBA-GEIGY) <br> * Page 2, ligne 20 - page 3, ligne 1; page 5, ligne 1 - page 8, ligne 11; page 19, exemple 26; revendications * | 1,5 | A 61 K  31/165 <br> A 61 K   9/16 |
| A | | 2-4 | |
|   | --- | | |
| Y | FR-A-2 187 312  (BLUM) <br> * Page 1, lignes 1-17; page 2, exemple 1; revendications 1,2 * | 1,5 | |
|   | --- | | |
| A | DE-A-2 416 903  (BAYER) <br><br> * Page 1, ligne 1 - page 4, ligne 22 * | | |
|   | --- | | **DOMAINES TECHNIQUES RECHERCHES (Int. Cl.4)** |
| A | CHEMICAL ABSTRACTS, vol. 101, no. 2, juillet 1984, page 294, no. 12086z, Columbus, Ohio, US; Y. KAWASHIMA et al.: "Development of spherical crystallization technique for a particle design method", & FUNTAI KOGAKU KAISHI 1983, 20(12), 759-62 * Résumé * | | A 61 K <br> B 01 J |
|   | ----- | | |

Le présent rapport de recherche a été établi pour toutes les revendications

| Lieu de la recherche | Date d'achèvement de la recherche | Examinateur |
|---|---|---|
| LA HAYE | 08-12-1986 | BENZ K.E. |